# EUROPEAN PATENT APPLICATION

(11) **EP 2 027 865 A1**
(43) Date of publication of application: **25.02.2009**
(21) Application number: 07743217.7
(22) Date of filing: 11.05.2007
(51) Int. Cl.: A61K 35/74, A61K 9/127, A61K 47/42, A61P 37/04

(54) **LIPOSOME HAVING LIPID MEMBRANE CONTAINING BACTERIAL CELL COMPONENT**

(30) Priority: 12.05.2006 JP 2006134526
(71) Applicant: National University Corporation Hokkaido University, Sapporo-shi Hokkaido 060-0808 (JP); Japan BCG Laboratory, Bunkyo-ku Tokyo 112-0006 (JP); University of Tsukuba, Tsukuba-shi, Ibaraki 305-8577 (JP)
(72) Inventor: ATTHACHAI, Homhuan, Kita 12-jyo Nishi 6-chome, Kita-ku Sapporo-shi Hokkaido 060-0812 (JP); KOGURE, Kentaro, Kita 12-1yo Nishi 6-chome, Kita-ku Sapporo-shi Hokkaido 060-0812 (JP); HARASHIMA, Hideyoshi, Kita 12-jyo Nishi 6-chome, Kita-ku Sapporo-shi Hokkaido 060-0812 (JP); YANO, Ikuya, Tokyo 204-0022 (JP); HATAKEYAMA, Yoshio, Tokyo 112-0006 (JP); YOKOMIZO, Hidehiro, 3-1-5, Matuyama, kiyose-shi Tokyo 204-0022 (JP); AKAZA, Hideyuki, Ibaraki-ken 305-8577 (JP)
(74) Representative: Adam, Holger
(86) International application number: PCT/JP2007/059782
(87) International publication number: WO 2007/132790

(57) **Abstract**

The object is to provide a liposome by which a bacterial cell component insoluble in water and an organic solvent (e.g., BCG-CW) can be pharmaceutically prepared in a form applicable to a living body (i.e., a form in which the bacterial cell component can be dispersed in an aqueous solvent and has a controlled size) without using any surfactant. Disclosed is a liposome having a lipid membrane containing a bacterial cell component insoluble in water and an organic solvent (e.g., BCG-CW).

## Description

### Technical Field

The present invention relates to a liposome having a lipid membrane containing a bacterial cell component which is insoluble in water and organic solvents, and a pharmaceutical composition containing the liposome.

### Background Art

Development of a vector or a carrier for delivering drugs, nucleic acids, peptides, proteins, sugars and the like surely to a target site is being actively carried out. For example, in gene therapy, viral vectors such as retroviruses, adenoviruses and adeno-associated viruses have been developed as vectors for introducing desired genes to target cells. However, viral vectors have problems such as difficulties in mass production, antigenicity and toxicity, and thus liposome vectors or peptide carriers, which have fewer of such problems, have been attracting attention. Liposome vectors also have an advantage that the directionality to a target site can be enhanced by introducing a functional molecule such as an antibody, a protein or a sugar chain, to the surface of a liposome vector.

Recently, it has been reported that when the surface of a condensed DNA-encapsulated liposome is modified with stearylated octaarginine, the cellular transfection efficiency for condensed DNA was enhanced by 1000 times, and the transfection efficiency of the condensed DNA-encapsulated liposome into cells was enhanced by 100 times (Patent Document 1, Non-Patent Documents 1 and 2). It has also been reported that a liposome having the surface modified with stearylated octaarginine can be transfected into cells while maintaining the original form (in an intact state) (Patent Document 1, Non-Patent Documents 1 and 2).

BCG (Mycobacterium bovis bacillus Calmette-Guerin, an attenuated bovine tuberculosis bacillus) is known to have a tumor reducing effect, in addition to the vaccinating effect against tuberculosis. It is conceived that this tumor reducing effect is a result of the manifestation of a so-called tumor immunotherapeutic effect, in which cellular immunity is activated by the strong adjuvant (immunostimulatory) action possessed by BCG.

However, the adverse side effects caused by administering live tubercle bacillus to human being have been an obstruction to the clinical application of BCG to tumor treatment. With regard to this problem of adverse side effects, a method of using a bacterial cellular component, for example, BCG-CW which is a cell wall fraction of BCG, as an adjuvant is attracting attention. However, since bacterial cellular components such as BCG-CW have special structures and physicochemical properties, the components are insoluble in water and organic solvents. Furthermore, since the bacterial cellular components have negative electric charges, the components have problems such as low affinity to host cellular membranes which have the same negative charge, and low efficiency of cellular uptake. Moreover, the bacterial cellular components such as BCG-CW need to be solubilized by surfactants, however, even in addition to the fact that adverse side effects such as stimulation by surfactants could be a problem, the problem that the efficiency of cellular uptake is still low because those bacterial cellular components solubilized by surfactants have large particle sizes (about 300 nm or more) and are negatively charged, is yet to be solved.
Patent Document 1: International Publication WO 2005/032593 pamphlet
Non-Patent Document 1: Kogure, et al., "Journal of Controlled Release," Vol. 98, pp. 317-323 (2004)
Non-Patent Document 2: Khalil Ikramy, et al., "Yakugaku Zasshi," Vol. 124, Suppl. 4, pp. 113-116 (2004)

### Disclosure of the Invention

### Problems to be Solved by the Invention

It is an object of the present invention to provide a liposome by which a bacterial cellular component insoluble in water and organic solvents (for example, BCG-CW) can be formulated into a form applicable to a living body (a form capable of being dispersed in aqueous solvents and well controlled in size) without making use of surfactants, and to provide a pharmaceutical composition containing the liposome.

### Means for Solving the Problems

In order to solve the above problems, the present invention provides a liposome having a lipid membrane containing a bacterial cellular component which is insoluble in water and organic solvents, and a pharmaceutical composition containing the liposome. According to the liposome of the present invention, a bacterial cellular component which is insoluble in water and organic solvents can be formulated into a form applicable to a living body (a form capable of being dispersed in an aqueous solvent, and well controlled in size), without making use of surfactants.

With regard to the liposome of the present invention, it is preferable that the bacterial cellular component is a cell wall fraction or cell wall skeleton fraction of a bacterium of genus Mycobacterium, a bacterium of genus Nocardia, a bacterium of genus Corynebacterium or a bacterium of genus Rhodococcus, and it is preferable that the bacterium of genus Mycobacterium is BCG.

The liposome of the present invention preferably has, on the liposome surface, a peptide containing a plurality of contiguous arginine residues. Thereby, the liposome of the present invention can be transferred into the inside of the host cells mainly through macropinocytosis, and thus a bacterial cellular component which is insoluble in water and organic solvents can be efficiently introduced into the cells.

With regard to the liposome of the present invention, it is preferable that the peptide is consisting of 4 to 35 amino acid residues including 4 to 20 contiguous arginine residues.

With regard to the liposome of the present invention, it is preferable that the peptide consists of arginine residues.

With regard to the liposome of the present invention, it is preferable that the amount of the peptide is 2% (molar ratio) or more based on the total lipids constituting the liposome.

With regard to the liposome of the present invention, it is preferable that the peptide is modified with a hydrophobic group or a hydrophobic compound, and the hydrophobic group or the hydrophobic compound is inserted into the lipid membrane forming the surface of the liposome, while the peptide is exposed from the lipid membrane forming the surface of the liposome.

With regard to the pharmaceutical composition of the present invention, it is preferable that the bacterial cellular component has immune activating potency, and the pharmaceutical composition is an immune activator.

### Effects of the Invention

According to the present invention, a liposome by which a bacterial cellular component insoluble in water and organic solvents (for example, BCG-CW) can be formulated into a form applicable to a living body (a form capable of being dispersed in aqueous solvents and well controlled in size) without making use of surfactants, and a pharmaceutical composition containing the liposome are provided.

### Brief Description of the Drawings

Fig. 1 is a diagram showing a structure of a functional domain included in BCG-CWS;
Fig. 2 is a diagram showing results of observation under a confocal laser microscope;
Fig. 3 is a diagram showing results of observation under a confocal laser microscope;
Fig. 4 shows results of an analysis of the amount of fluorescence in cells employing flow cytometry of bladder cancer cell line MBT-2 which has taken up an R8-modified BCG liposome;
Fig. 5 shows an intracellular localizability of the R8-modified BCG liposome taken up into MBT-2. The blue color on the upper left side represents the light emission by Hoechst 33342^{™}; the green color on the upper right side represents the light emission by R8-modified BCG liposome; the red color on the lower left side represents the light emission by LysotrackerRed^{™}; and the lower right side represents superposition of the three types of light emission; and
Fig. 6 is a diagram showing results for a measurement of activation of dendritic cells, using CD86, CD80 and MHCH as indices.

### Best Mode for Carrying Out the Invention

The liposome of the present invention is a closed vesicle having a lipid membrane (lipid, bylayer membrane), and the number of lipid membranes possessed by the liposome of the present invention is not particularly limited. The liposome of the present invention may be a multilamellar liposome (MLV), or may also be a unilamellar liposome such as an SUV (small unilamellar vesicle), an LUV (large unilamellar vesicle) or a GUV (giant unilamellar vesicle). The size of the liposome of the present invention is not particularly limited, but it is preferably 50 to 300 nm in diameter, and more preferably 100 to 200 nm in diameter.

Examples of the constituent components of the lipid membrane include lipids, membrane stabilizers, antioxidants, charged substances, membrane proteins, and the like.

The lipids are essential constituent components of the lipid membrane, and the amount of lipids included in the lipid membrane is usually 70% (molar ratio) or more, preferably 75% (molar ratio) or more, and more preferably 80% (molar ratio) or more, of the total amount of the materials constituting the lipid membrane. In addition, the upper limit of the amount of lipids contained in the lipid membrane is 100% of the total amount of the materials constituting the lipid membrane.

As for the lipids, for example, the phospholipids, glycolipids, sterols, saturated or unsaturated fatty acids and the like exemplified in the following may be mentioned.

### [Phospholipids]

Phosphatidylcholines (for example, dioleoylphosphatidylcholine, dilauroylphosphatidylcholine, dimyristoylphosphatidylcholine, dipalmitoylphosphatidylcholine, distearoylphosphatidylcholine, and the like), phosphatidylglycerols (for example, dioleoylphosphatidylglycerol, dilauroylphosphatidylglycerol, dimyristoylphosphatidylglycerol, dipalmitoylphosphatidylglycerol, distearoylphosphatidylglycerol, and the like), phosphatidylethanolamines (for example, dilauroylphosphatidylethanolamine, dimyristoylphosphatidylethanolamine, dipalmitoylphosphatidylethanolamine, distearoylphosphatidylethanolamine, and the like), phosphatidylserine, phosphatidylinositol, phosphatidic acid, cardiolipin, sphingomyelin, ceramide phosphorylethanolamine, ceramide phosphorylglycerol, ceramide phosphorylglycerol phosphate, 1,2-dimyristoyl-1,2-deoxyphosphatidylcholine, plasmalogen, egg yolk lecithin, soybean lecithin, hydrogenation products thereof, and the like.

### [Glycolipids]

Glyceroglycolipids (for example, sulfoxyribosyl glyceride, diglycosyl diglyceride, digalactosyl diglyceride, galactosyl diglyceride, and glycosyl diglyceride), sphingoglycolipids (for example, galactosyl cerebroside, lactosyl cerebroside, and ganglioside), and the like.

### [Sterols]

Animal-derived sterols (for example, cholesterol, cholesterol succinate, cholestanol, lanosterol, dihydrolanosterol, desmosterol, and dihydrocholesterol), plant-derived sterols (phytosterols) (for example, stigmasterol, sitosterol, campesterol, and brassicasterol), microbial-derived sterols (for example, zymosterol and ergosterol), and the like.

### [Saturated or unsaturated fatty acids]

Saturated or unsaturated fatty acids having 12 to 20 carbon atoms, such as palmitic acid, oleic acid, stearic acid, arachidonic acid, myristic acid, and the like.

The membrane stabilizer is an optional constituent component of the lipid membrane, which can be included so as to physically or chemically stabilize the lipid membrane, or to control the fluidity of the lipid membrane. The amount of the membrane stabilizer included in the lipid membrane is usually 30% (molar ratio) or less, preferably 25% (molar ratio) or less, and more preferably 20% (molar ratio) or less, of the total amount of the materials constituting the lipid membrane. In addition, the lower limit of the content of the membrane stabilizer is zero.

As for the membrane stabilizer, for example, sterols, glycerin or fatty acid esters thereof, and the like may be mentioned. As for the sterol, specific examples as described above may be mentioned, and as for the fatty acid esters of glycerin, for example, triolein, trioctanoin and the like may be mentioned.

The antioxidant is an optional constituent component of the lipid membrane which can be included to prevent oxidation of the lipid membrane, and the amount of the antioxidant included in the lipid membrane is usually 30% (molar ratio) or less, preferably 25% (molar ratio) or less, and more preferably 20% (molar ratio) or less, of the total amount of the materials constituting the lipid membrane. In addition, the lower limit of the content of the antioxidant is zero.

As for the antioxidant, for example, tocopherol, propyl gallate, ascorbyl palmitate, butylated hydroxytoluene, and the like may be mentioned.

The charged substance is an optional constituent component of the lipid membrane which can be included to impart a positive charge or a negative charge to the lipid membrane, and the amount of the charged substance included in the lipid membrane is usually 30% (molar ratio) or less, preferably 25% (molar ratio) or less, and more preferably 20% (molar ratio) or less, of the total amount of the material constituting the lipid membrane. In addition, the lower limit of the content of the charged substance is zero.

As for the charged substance imparting a positive charge, for example, saturated or unsaturated aliphatic amines such as stearylamine and oleylamine, saturated or unsaturated cationic synthetic lipids such as dioleoyltrimethylammoniumpropane, and the like may be mentioned, and as for the charged substances imparting a negative charge, for example, dicetyl phosphate, cholesteryl hemisuccinate, phosphatidylserine, phosphatidylinositol, phosphatidic acid and the like may be mentioned.

The membrane protein is an optional constituent component of the lipid membrane which can be included to maintain the structure of the lipid membrane or to impart functionality to the lipid membrane, and the amount of the membrane protein included in the lipid membrane is usually 10% (molar ratio) or less, preferably 5% (molar ratio) or less, and more preferably 2% (molar ratio) or less, of the total amount of the materials constituting the lipid membrane. In addition, the lower limit of the content of the membrane protein is zero.

Examples of the membrane protein include peripheral membrane proteins, integral membrane proteins and the like.

As the lipid constituting the lipid membrane, lipid derivatives having a blood retentive function, temperature change sensitive function, a pH sensitive function or the like, may also be used. Thereby, one or two or more functions among the above-mentioned functions can be imparted to the liposome. By imparting a blood retentive function to the liposome, the retentivity of the liposome in the blood is improved, so that the rate of capture by the tissues of the reticuloendothelial system such as liver and spleen can be decreased. By imparting a temperature change sensitive function and/or a pH sensitive function to the liposome, the releasability of a target substance encapsulated in the liposome can be increased.

Examples of the blood retentive lipid derivative which can impart the blood retentive function include glycophorin, ganglioside GM1, phosphatidylinositol, ganglioside GM3, glucuronic acid derivatives, glutamic acid derivatives, polyglycerophospholipid derivatives; polyethylene glycol derivatives such as N-{carbonyl-methoxypolyethylene glycol-2000}-1,2-dipalmitoyl-sn-glycero-3-phosphoethanolamine,
N-{carbonyl-methoxypolyethylene
glycol-5000}-1,2-dipalmitoyl-sn-glycero-3-phosphoethanolamine,
N- {carbonyl-methoxypolyethylene
glycol-750}-1,2-distearoyl-sn-glycero-3-phosphoethanolamine,
N- {carbonyl-methoxypolyethylene
glycol-2000}-1,2-distearoyl-sn-glycero-3-phosphoethanolamine,
N- {carbonyl-methoxypolyethylene glycol-5000}-1,2-distearoyl-sn-glycero-3-phosphoethanolamine; and the like.

Examples of the temperature change sensitive lipid derivative which can impart the temperature change sensitive function include dipalmitoylphosphatidylcholine and the like, while examples of the pH sensitive lipid derivative which can impart the pH sensitive function include dioleoylphosphatidylethanolamine and the like.

The liposome of the present invention has a lipid membrane containing a bacterial cellular component which is insoluble in water and organic solvents. If the liposome is a unilamellar liposome, the bacterial cellular component is included in the unilamellar lipid membrane possessed by the liposome, and if the liposome is a multilamellar liposome, the bacterial cellular component is included in one or two or more lipid membranes among the plurality of lipid membranes possessed by the liposome. The amount of the bacterial cell component contained in the lipid membrane is usually 1 to 15% (weight ratio), preferably 2 to 10% (weight ratio), and more preferably 3 to 7% (weight ratio), of the total amount of the materials constituting the lipid membrane.

The bacterial cellular component is not particularly limited as long as the material is insoluble in water and organic solvents (for example, chloroform, dichloroacetic acid, methanol, ethanol, diethyl ether, hexane, tetrahydrofuran and the like), and for example, there may be mentioned a cell wall fraction or cell wall skeleton fraction of a bacterium of genus Mycobacterium, a bacterium of genus Nocardia, a bacterium of genus Corynebacterium, a bacterium of genus Rhodococcus, or the like.

The cell wall of the bacteria of genus Mycobacterium contains a polymer comprising mycolic acid (fatty acid), arabinogalactan (polysaccharide) and peptidoglycan, and the fundamental structure of the cell wall is composed of this polymer. This fundamental structure is referred to as "cell wall skeleton (CWS)." Bacteria of genus Nocardia, bacteria of genus Corynebacterium, bacteria of genus Rhodococcus and the like, which are related to the bacteria of genus Mycobacterium in terms of classification, also have the same cell wall skeleton as that of the bacteria of genus Mycobacterium.

The cell wall(CW) fraction is not particularly limited in the composition, the method of preparation or the like, as long as the fraction contains the cell wall skeleton(CWS) as the main component, but for example, the cell wall fraction can be prepared by the following method. Heat-killed bacterial cells are suspended in a ten-fold volume of purified water, and the cells are disrupted homogenously by a French press treatment. Subsequently, the cell homogenate is centrifuged at 6760 × g to remove any unbroken cells, and the centrifugation supernatant is further centrifuged at 18000 × g so that a cell wall fraction can be obtained as a sediment. The median diameter of micelles formed by suspending the cell wall fraction (for example, BCG-CW) in water, a buffer solution or the like, is usually 120 to 320 nm, preferably 170 to 270 nm, and more preferably 220 nm. In addition, the median diameter is measured with, for example, a particle size distribution analyzer.

The cell wall skeleton fraction(CWS) is a fraction that is obtained by purifying from the crude cell wall fraction, and for example, can be prepared by the following method. A cell wall fraction is suspended in a phosphate buffer solution, and cell wall-bound nucleic acids (for example, oligoDNA) are decomposed and removed by a DNase treatment, and at the same time, cell wall-bound proteins are decomposed and removed by a pronase and trypsin treatment. Subsequently, lipids are extracted and removed by an extraction treatment with an organic solvent (for example, tetrahydrofuran). Thereby, the cell wall skeleton fraction can be obtained as a residue which is insoluble in water and organic solvents after those treatments. The median diameter of micelles formed by suspending the cell wall skeleton fraction (for example, BCG-CWS) in water, a buffer solution or the like, is usually 80 to 180 nm, preferably 100 to 150 nm, and more preferably 130 nm. In addition, the median diameter is measured with, for example, a particle size distribution analyzer.

The cell wall fraction(CW) or cell wall skeleton fraction(CWS) of a bacterium of genus Mycobacterium, a bacterium of genus Nocardia, a bacterium of genus Corynebacterium, a bacterium of genus Rhodococcus or the like; is insoluble in water and organic solvents, and also has immuno activating potency (adjuvant activity, immunostimulatory action). From the cell wall skeleton, the peptidoglycan part and the mycolic acid part are important in relation to the immuno activating action. The components other than the cell wall skeleton (for example, lipomannan, trehalose mycolate), which are contained in the cell wall fraction, also contribute to the immuno activating action.

As the bacteria of genus Mycobacterium, for example, the Mycobacterium tuberculosis complex including M. tuberculosis, M. bovis, M. africanum, M. microti, M. canettii, M. bovis BCG and the like may be mentioned.

As the bacteria of genus Nocardia, for example, N. asteroides, N. brasiliensis, N. rubra and the like may be mentioned.

As the bacteria of genus Corynebacterium, for example, C. diphtheriae, C. ulcerans and the like may be mentioned.

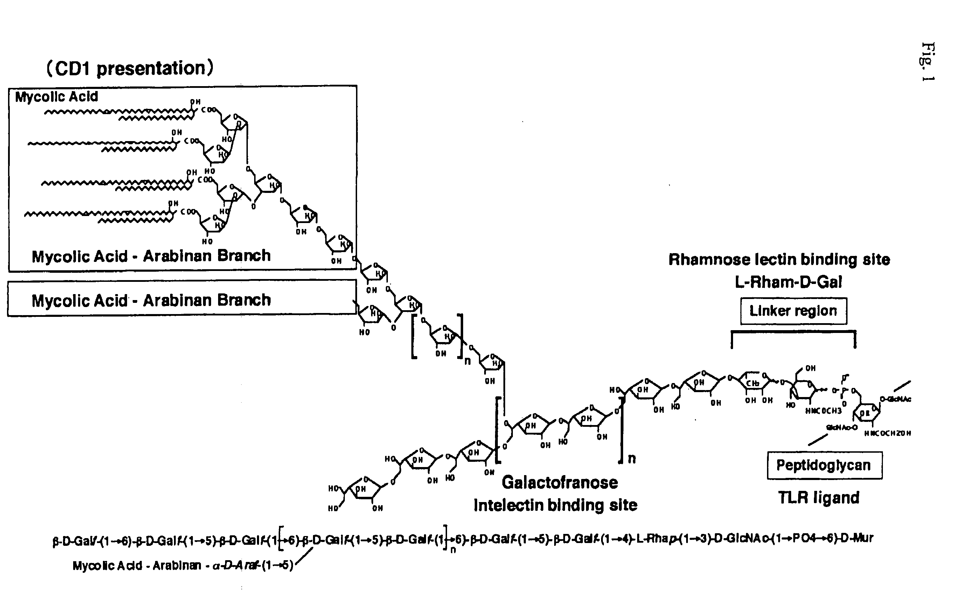

As for the bacterial cell component, it is preferable to use the cell wall fraction of BCG (BCG-CW) or the cell wall skeleton fraction thereof (BCG-CWS). BCG is an attenuated bacterium produced by repeating laboratory culture of Mycobacterum bovis, and is virtually non-pathogenic to human being. The functional domains included in BCG-CW or BCG-CWS include, in addition to the construct shown in Fig. 1, lipomannan (LM), lipoarabinomannan (LAM), diphosphatidylinositol (PIM2) or hexamannoside (PIM6) and cord factor (TDM).

The liposome of the present invention preferably has a peptide containing a plurality of contiguous arginine residues on the liposome surface. The number of contiguous arginine residues is not particularly limited as long as it is plural, but the number is usually from 4 to 20, preferably from 6 to 12, and more preferably from 7 to 10. The total number of amino acid residues constituting the peptide is not particularly limited, but the total number is usually from 4 to 35, preferably from 6 to 30, and more preferably from 7 to 23. The peptide may contain an arbitrary amino acid sequence at the C-terminal and/or N-terminal of the plurality of contiguous arginine residues, but it is preferable that the peptide consists of arginine residues. The amino acid sequence added to the C-terminal or N-terminal of the plurality of contiguous arginine residues is preferably an amino acid sequence having rigidity (for example, polyproline). Unlike polyethylene glycol (PEG) which takes a flexible and irregular form, polyproline is linear and maintains stiffness to a certain degree. Furthermore, the amino acid residues included in the amino acid sequence which is added to the C-terminal or N-terminal of the plurality of contiguous arginine residues, are preferably amino acid residues other than acidic amino acids. This is because there is a possibility that acidic amino acid residues having negative charges electrostatically interact with arginine residues having positive charges, thus attenuating the effects of the arginine residues.

The amount of the peptide present on the liposome surface is usually 0.1 to 30% (molar ratio), preferably 0.2 to 25% (molar ratio), and more preferably 0.5 to 20% (molar ratio), based on the total lipids constituting the liposome.

In the case where a peptide containing a plurality of contiguous arginine residues is present on the liposome surface, the liposome of the present invention can be transferred into cells through the peptide present on the surface (see WO 2005/032593). When the amount of the peptide present on the liposome surface is less than 2% (molar ratio), preferably less than 1.5% (molar ratio), and more preferably less than 1% (molar ratio), based on the total lipids constituting the liposome, the liposome can be transferred into cells mainly by endocytosis (see WO 2005/032593). The lower limit of the amount of peptide in this case is usually 0.1% (molar ratio), preferably 0.5% (molar ratio), and more preferably 0.7% (molar ratio), based on the total lipids constituting the liposome. On the other hand, when the amount of the peptide present on the liposome surface is 2% (molar ratio) or more, preferably 3% (molar ratio) or more, and more preferably 4% (molar ratio) or more, based on the total lipids constituting the liposome, the liposome can be transferred into cells mainly by macropinocytosis (see WO 2005/032593). The upper limit of the amount of peptide in this case is usually 30% (molar ratio), preferably 25% (molar ratio), and more preferably 20% (molar ratio), based on the total lipids constituting the liposome.

In macropinocytosis, an extracellular material is taken up into a cell as a fraction called macropinosome, and since the macropinosome does not fuse with lysosomes, unlike endosomes, the material encapsulated in the macropinosome can avoid decomposition by lysosomes. Therefore, in the case where a liposome is transferred into cells by macropinocytosis, the liposome can be efficiently transferred into cells.

If the intracellular transfer path of the liposome depends on endocytosis, the lipid membrane is required to include cationic lipids as main components. However, if the intracellular transfer path of the liposome does not depend on endocytosis, there is no need for cationic lipids to be included in the lipid membrane, and the cytotoxicity caused by cationic lipids can be reduced. The ratio of cationic lipids relative to the total lipids constituting the liposome is preferably 0 to 40% (molar ratio), and more preferably 0 to 20% (molar ratio).

Examples of the cationic lipids include DODAC (dioctadecyldimethylammonium chloride), DOTMA (N-(2,3-dioleyloxy)propyl-N,N,N-trimethylammonium), DDAB (didodecylammonium bromide), DOTAP (1,2-dioleoyloxy-3-trimethylammonio propane), DC-Chol (3β-N-(N',N'-dimethylaminoethane)-carbarnoyl cholesterol), DMRIE (1,2-dimyristoyloxypropyl-3-dimethylhydroxyethylammonium), DOSPA (2,3-dioley-loxy-N-[2(sperminecarboxamido)ethyl]-N,N-dimethyl-1--propanaminum trifluoroacetate) and the like. Examples of non-cationic lipids (neutral lipids or anionic lipids) include neutral lipids such as diacylphosphatidylcholine, diacylphosphatidylethanolamine, cholesterol, ceramides, sphingomyelin, cephalin and cerebrosides; and anionic lipids such as cardiolipin, diacylphosphatidylserine, diacylphosphatidic acid, N-succinylphosphatidylethanolamine (N-succinyl PE), phosphatidic acid, phosphatidylinositol, phosphatidylglycerol, phosphatidylethylene glycol, and cholesterol succinate.

It is preferable that the peptide containing a plurality of contiguous arginine residues is modified with a hydrophobic group or a hydrophobic compound, and the peptide is present on the liposome surface such that the hydrophobic group or hydrophobic compound is inserted into the lipid membrane forming the surface of the liposome, while the peptide is exposed from the lipid membrane forming the surface of the liposome.
When the peptide containing a plurality of contiguous arginine residues exists on the liposome surface while maintaining certain orientation, the liposome can be efficiently transferred into cells by macropinocytosis.

The hydrophobic group or hydrophobic compound is not particularly limited as long as the group or compound can be inserted into the lipid membrane. The lipid membrane consists of a hydrophilic part and a hydrophobic part, but the hydrophobic group or hydrophobic compound is inserted into the lipid membrane in a state of being bound by hydrophobic bonding with the hydrophobic part of the lipid membrane. As for the hydrophobic group, for example, a saturated or unsaturated fatty acid group such as a stearyl group, a cholesterol group or a derivative thereof, and the like may be mentioned, and among these, particularly a fatty acid group having 10 to 20 carbon atoms (for example, a palmitoyl group, an oleoyl group, a stearyl group, an arachidonoyl group, and the like) is preferred. Furthermore, as for the hydrophobic compound, for example, the phospholipids, glycolipids or sterols exemplified in the above, long-chain aliphatic alcohols (for example, phosphatidylethanolamine, cholesterol and the like), polyoxypropylenealkyls, glycerol fatty acid esters, and the like may be mentioned.

A preparation example of liposome according to a hydration method will be described in the following.
In an organic solvent, lipids which are constituent components of lipid membrane, a predetermined bacterial cell component, and a predetermined peptide modified with a hydrophobic group or a hydrophobic compound are mixed, and the organic solvent is removed by evaporation, to thus obtain a lipid membrane. At this time, as the organic solvent, for example, hydrocarbons such as pentane, hexane, heptane and cyclohexane; halogenated hydrocarbons such as methylene chloride and chloroform; aromatic hydrocarbons such as benzene and toluene; lower alcohols such as methanol and ethanol; esters such as methyl acetate and ethyl acetate; ketones such as acetone; and the like may be mentioned, and these may be used individually alone or in combination of two or more species. Subsequently, when the lipid membrane is hydrated and stirred or ultrasonicated, a liposome provided with a lipid membrane containing a predetermined bacterial cell component, and having a predetermined peptide on the surface can be prepared.

Another production example according to the hydration method will be described in the following.
In an organic solvent, lipids which are constituent components of lipid membrane, and a predetermined bacterial cell component are mixed, and the organic solvent is removed by evaporation, to form thus a lipid membrane. As this lipid membrane is hydrated, and stirred or ultrasonicated, a liposome with a lipid membrane containing an isolated bacterial cellular component is prepared. Subsequently, a predetermined peptide modified with a hydrophobic group or a hydrophobic compound is added to the external solution of this liposome, to thereby introduce the predetermined peptide on the liposome surface.

By passing the liposome through a membrane filter having a predetermined pore size, a liposome having a certain particle size distribution can be obtained. Further, conversion from a multilamellar liposome to a unilamellar liposome, and conversion from a unilamellar liposome to a multilamellar liposome can be carried out according to known methods.

By means of the liposome provided with a lipid membrane containing a bacterial cellular component which is insoluble in water and organic solvents, the bacterial cellular component can be formulated into a form applicable to the living body (form which is dispersible in aqueous solvents and is controlled in size), without using surfactants. Also, when a liposome having a peptide containing a plurality of contiguous arginine residues on the liposome surface is used, the bacterial cellular component can be efficiently introduced into cells. The type of cells to which the bacterial cellular component should be introduced is not particularly limited. For example, dendritic cells, macrophages, T lymphocytes, monocytes, polymorphonuclear leukocytes, tumor cells and the like may be mentioned.

The liposomal formulation of the present invention containing particular composition of bacterial cellular component can be used in pharmaceutical uses in accordance with the action of the bacterial cellular component. For example, in the case where the bacterial cellular component has immuno potentiating action, the pharmaceutical composition containing the liposome of the present invention can be used as an immuno activator (adjuvant). An immuno activator can activate humoral immunity (antibody production) and cell-mediated immunity sensitized T limphocyte production, by activating immunocytes such as dendritic cells (antigen presenting cells). The immuno activator may activate immunocytes by being taken up into the immunocytes, or may also activate the immunocytes by exerting action from the outside of the immunocytes.

As for the dosage form of the pharmaceutical composition, for example, a dispersion of liposome or a dried product thereof (for example, freeze-dried product, spray-dried product, or the like) may be mentioned. As the dispersion solvent, for example, buffer solutions such as physiological saline, phosphate buffer solution, citrate buffer solution and acetate buffer solution can be used. The dispersion may be added with additives such as, for example, a sugar, a polyhydric alcohol, a water-soluble polymer, a nonionic surfactant, an antioxidant, a pH adjusting agent and a hydration promoting agent, and used.

The pharmaceutical composition can be used in vivo as well as in vitro. In the case of using the pharmaceutical composition in vivo, the administration route may be exemplified by parenteral administration routes such as intravenous, intraperitoneal, subcutaneous intradermal and intranasal routes. The dosage and administration frequency can be appropriately controlled in accordance with the type or amount of the bacterial cell component, target organs, or the like.

### EXAMPLES

### [Example 1]

A chloroform solution of lipids (egg yolk phosphatidylcholine (EPC)/cholesterol (Chol) = 7:3 (molar ratio)) (amount of lipid: 10 µmol), FTSC (Fluorescein-5-thiosemicarbazide, green)-labeled BCG-CW suspended in chloroform/ethanol (2:1) (amount of BCG-CW: 0.4 mg), and an aqueous solution of stearyl octaarginine (STR-R8) (amount of STR-R8: 0.8 mol% of the amount of lipids) are mixed, and the mixture is evaporated to dryness under reduced pressure in a rotary evaporator, to thereby prepare a lipid thin film containing BCG-CW and STR-R8. To the resulting lipid thin film, 1 mL of a buffer solution (5 mM HEPES, 0.15 M NaCl, pH 7.4) was added to hydrate the film, and then by adding glass beads and rotating the rotary evaporator at a typical atmospheric pressure (no pressure reduction) at 65°C for 20 minutes, a liposome suspension was prepared. In addition, 1 mM Rhodamine (red) was dissolved in the buffer solution for hydrating the lipid thin film, as an aqueous phase marker of the liposome.
The resulting liposome suspension was passed several times through a membrane of 0.8 µm, 0.4 µm or 0.1 µm (extruder treatment)pore, respectively, and thus a liposome having a small particle diameter (about 120 nm) was prepared. This liposome has a lipid membrane containing BCG-CW and has STR-R8 on the surface (hereinafter, referred to as "R8-modified BCG liposome").

In addition, the BCG-CW used herein was a product manufactured by Japan BCG Laboratory using the following method. Heat-killed cells of BCG were suspended in a 10-fold volume of purified water, and the cells were homogenized by a French press treatment. Subsequently, the cell homogenate was centrifuged at 6760 × g to exclude unbroken cells, and the supernatant was centrifuged at 18000 × g to obtain a time sediment. This fine sediment was freeze-dried to obtain BCG-CW The median diameter of micelles formed by suspending the BCG-CW thus obtained in purified water or the like, is usually 120 to 320 nm, preferably 170 to 270 nm, and more preferably 220 nm, when measured with a particle size distribution analyzer.

A chloroform solution of lipids (EPC/Chol = 7:3 (molar ratio)) (amount of lipids: 10 µmol), and FTSC-labeled BCG-CW suspended in chloroform/ethanol (2:1) (amount of BCG-CW: 0.4 mg) were mixed, and the mixture was dried to solid under reduced pressure in a rotary evaporator, to thus prepare a lipid thin film which contains BCG-CW or BCG-CWS but does not contain STR-R8. Then, the lipid thin film was treated as described above, to prepare a liposome which has a lipid membrane containing BCG-CW or BCG-CWS but does not have STR-R8 on the surface (hereinafter, referred to as "R8-unmodified BCG liposome").

Under observation with a confocal laser microscope, NIH3T3 cells (2.5 × 10⁵ cells/60 mm dish) were incubated overnight in 10% FBS-containing DMEM. Subsequently, the NIH3T3 cells were incubated in a non-serum-containing DMEM culture solution containing R8-modified or unmodified BCG liposome (final lipid concentration: 50 µM). The incubation was carried out at 37°C for 1 hour. Upon completion of the incubation, the cells were washed and then observed under a confocal laser microscope without fixing.

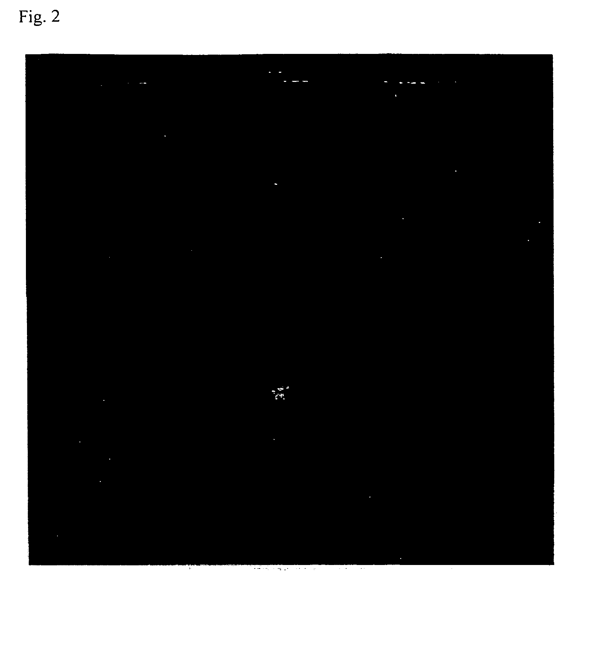

As a result, as shown in Fig. 2, in the case of the R8-unmodified BCG liposome, green color (FTSC-labeled BCG-CW contained in the lipid membrane of the liposome) and red color (Rhodamine as an aqueous phase marker of the liposome) were virtually not observed in the cells. This implies that the uptake amount of R8-unmodified BCG liposome into cells is low. It is conceived that since BCG-CW or BCG-CWS is negatively charged, the liposome surface is also negatively charged (zeta potential: -2.94 mV), and repels from the cellular membrane surface having a negative charge, and thus the uptake amount into cells is low.

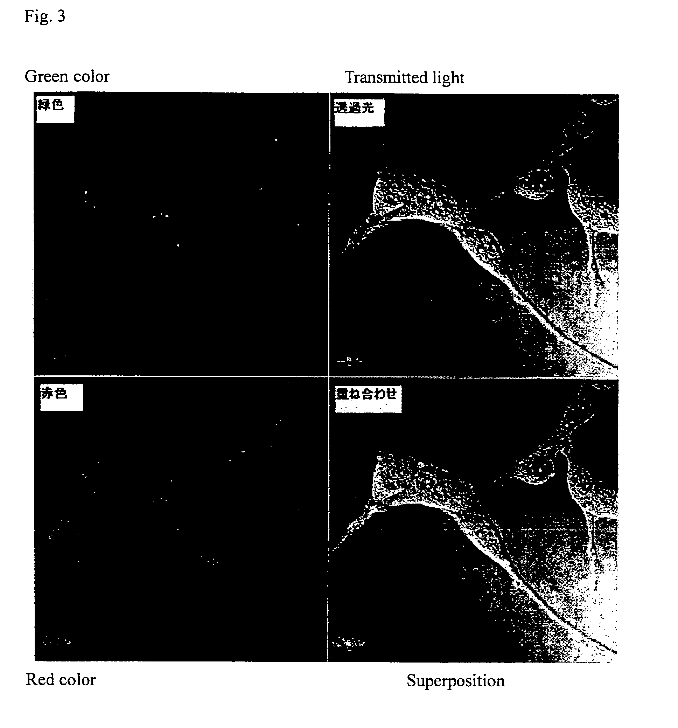

Meanwhile, as shown in Fig. 3, in the case of the R8-modified BCG liposome, green color (FTSC-labeled BCG-CW contained in the lipid membrane of the liposome) and red color (Rhodamine as an aqueous phase marker of the liposome) were observed in the cells. This implies that the uptake amount of R8-modified BCG liposome into cells is high. It is conceived that since the liposome surface is positively charged due to the STR-R8 modification (zeta potential: +25.59 mV), the uptake amount into cells is high.
In addition, any morphological changes in the cells due to the R8-unmodified BCG liposome were not recognized.

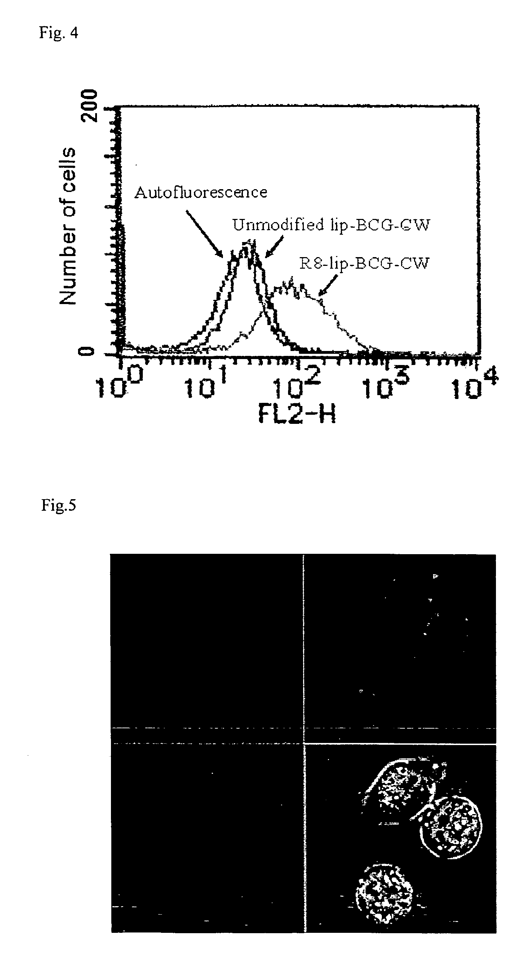

Subsequently, to 5 × 10⁵ cells of bladder cancer cell line MBT-2 (RIKEN) cultured using an RPMI1640 medium (Sigma-Aldrich Company) added with 10% fetal bovine serum at 5% CO₂, the R8-unmodified BCG liposome or R8-modified BCG liposome were added in an amount of 3.3 mg each, and culture was carried out at 37°C for 1 hour. The intracellular fluorescence level was analyzed using flow cytometry (BD Biosciences, Inc.), and since the intracellular fluorescence level increased for the R8-modified BCG liposome, an increase in the intracellular uptake amount due to the STR-8 modification was confirmed (Fig. 4). Furthermore, 30 minutes before the visualization of fluorescence, the endosome/lysosome and the nucleus were treated to emit red light and blue right, respectively using 75 nM LysotrackerRed^{™} and Hoechst33342^{™}. The R8-modified BCG liposome (emitting green light) taken up into MBT-2 was localized in the endosome/lysosome fraction (Fig. 5).

### [Example 2]

A chloroform solution of lipids (EPC/Chol = 7:3 (molar ratio)) (amount of lipids: 10 µmol), FTSC-labeled BCG-CW suspended in chloroform/ethanol (2:1) (amount of BCG-CW: 0.4 mg), and an aqueous solution of STR-R8 (amount of STR-R8: 0.8 or 5 mol% of the amount of lipids) were mixed, and the mixture was evaporated to dryness under reduced pressure in a rotary evaporator, to thus prepare a lipid thin film containing BCG-CW and STR-R8. The lipid thin film was treated in the same manner as in Example 1, and thus a liposome having a lipid membrane containing BCG-CW and having STR-R8 on the surface was prepared (the liposome in which the amount of STR-R8 is 0.8 mol% of the amount of lipids is hereinafter referred to as "low density R8-modified BCG liposome", the liposome with the amount being 5 mol% is hereinafter referred to as "high density R8-modified BCG liposome").

A chloroform solution of lipids (EPC/Chol = 7:3 (molar ratio)) (amount of lipids: 10 µmol), and FTSC-labeled BCG-CW suspended in chloroform/ethanol (2:1) (amount of BCG-CW: 0.4 mg) were mixed, and the mixture was dried to solid under reduced pressure in a rotary evaporator, to thus prepare a lipid thin film which contained BCG-CW but did not contain STR-R8. The lipid thin film was treated in the same manner as in Example 1, and thus a liposome having a lipid membrane containing BCG-CW and not having STR-R8 on the surface was prepared (hereinafter, referred to as "R8-unmodified BCG liposome").

A chloroform solution of lipids (EPC/Chol = 7:3 (molar ratio)) (amount of lipids: 10 µmol), and an aqueous solution of STR-R8 (amount of STR-R8: 0.8 mol% of the amount of lipids) were mixed, and the mixture was evaporated to dryness under reduced pressure in a rotary evaporator, to thus prepare a lipid thin film which contained STR-R8 but did not contain BCG-CW. The lipid thin film was treated in the same manner as in Example 1, and thus a liposome having a lipid membrane not containing BCG-CW and having STR-R8 on the surface was prepared (hereinafter, referred to as "low density R8-modified, non-BCG-containing liposome").

A chloroform solution of lipids (EPC/Chol = 7:3 (molar ratio)) (amount of lipids: 10 µmol) was evaporated to dryness under reduced pressure in a rotary evaporator, to thus prepare a lipid thin film containing neither BCG-CW nor STR-R8. The lipid thin film was treated in the same manner as in Example 1, and thus a liposome having a lipid membrane not containing BCG-CW and not having STR-R8 on the surface was prepared (hereinafter, referred to as "R8-unmodified, non-BCG-containing liposome").

In a non-serum-containing culture solution of dendritic cells (DC) isolated from mouse bone marrow, various liposomes were added, and the cells were incubated at 37°C for 2 hours, and then cultured in the presence of serum for 22 hours. Then, the presence or absence of the activation of DC was examined. As indicated for the activation of DC, peripheral marker proteins CD86 and CD80, and MHCII related to antigen presentation were selected, and the amounts of marker proteins in the respective treated groups were measured by means of a flow cytometer (FACS), using the respective fluorescence-labeled antibodies for the proteins.

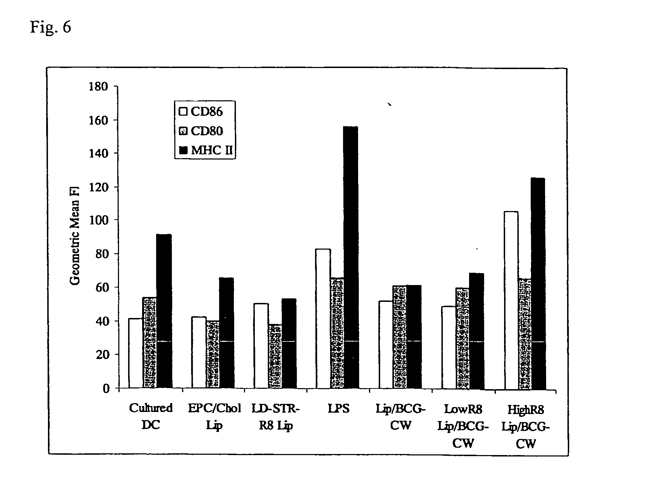

The results are presented in Fig. 6. In Fig. 6, "Cultured DC" represents the results of untreated dendritic cells, "EPC/Chol Lip" represents those of dendritic cells treated with the R8-unmodified, non-BCG-containing liposome, "LD-STR-R8 Lip" represents those of dendritic cells treated with the low density R8-modified, non-BCG-containing liposome, "LPS" represents those of dendritic cells treated with lipopolysaccharide, which is a representative immunocyte activating component (adjuvant) (positive control), "Lip/BCG-CW" represents those of dendritic cells treated with the R8-unmodified BCG liposome, "LowR8 Lip/BCG-CW" represents those of dendritic cells treated with the low density R8-modified BCG liposome, and "HighR8 Lip/BCG-CW" represents those of dendritic cells treated with the high density R8-modified BCG liposome.
As shown in Fig. 5, the high density R8-modified BCG liposome exhibited high immunocyte activation ability to the same level as that of LPS which is a representative adjuvant.

### [Example 3]

Using RPMI1640 medium (Sigma-Aldrich Company) added with 10% fetal bovine serum, bladder cancer cell line MBT-2 (RIKEN) was cultured at 37°C at 5% CO₂. Seven-week old C3H/HeN mice (Charles River Laboratories, Inc.) were divided into Groups A to G, and 0.7 × 10⁷ cells of MBT-2 and the following respective samples were transplanted on the right dorsal side of each mouse in the respective groups.

Group A: 100 µl of PBS buffer solution (6 animals)
Group B: 1 mg of BCG (Japan BCG Group)/100 µl of PBS buffer solution (6 animals)
Group C: 1 mg of BCG-CW prepared in Example 1/100 µl of PBS buffer solution (18 animals)
Group D: 0.1 mg of BCG-CW prepared in Example 1/100 µl of PBS buffer solution (6 animals)
Group E: R8-modifed, high BCG content liposome having a lipid membrane containing 1 mg of BCG-CW prepared in Example 1, and having STR-R8 on the surface/100 µl of PBS buffer solution (18 animals)
Group F: R8-modified, low BCG content liposome having a lipid membrane containing 0.1 mg of BCG-CW prepared in Example I, and having STR-R8 on the surface/100 µl of PBS buffer solution (6 animals)
Group G: R8-unmodified, non-BCG-containing liposome having a lipid membrane which does not contain BCG-CW, and not having STR-R8 on the surface/100 µl of PBS buffer solution (6 animals)

The above-mentioned liposomes were prepared by the method described in Example 2 for preparing a low density R8-modified BCG liposome, while changing the content of BCG-CW. Also, the R8-unmodified, non-BCG-containing liposome was prepared in the same manner as in the method described in Example 2.

After breeding the mice in the respective groups for 4 weeks, the tumor tissues derived from the transplanted cancer cells were extracted, and the size (mm²) was measured. Thus, the tumor reducing effects of the respective samples were examined. The results are presented in Table 1.

**[Table 1]**

| | Mice retaining tumor (animals) | | Size of tumor (mm²) | |
|---|---|---|---|---|
| Group | After 2 weeks of transplantation | After 4 weeks of Transplantation | Average | SD |
| A | 6/6 | 6/6 | 290.3 | 193.2 |
| B | 6/6 | 3/6 | 5.6 | 7.4 |
| C | 16/18 | 8/18 | 25.7 | 50.3 |
| D | 6/6 | 6/6 | 202.0 | 136.1 |
| E | 15/18 | 15/18 | 21.7 | 21.0 |
| F | 5/6 | 0/6 | 0.0 | 0.0 |
| G | 5/6 | 5/6 | 358.7 | 267.5 |

A tumor reducing effect similar to that of Group B (1 mg of BCG) and Group C (1 mg of BCG-CW) was recognized in Group E (R8-modified, high BCG content liposome). On the other hand, although no tumor reducing effect was recognized in Group D (0.1 mg of BCG-CW), Group F (R8-modified, low BCG content liposome) which contained the same amount of BCG-CW was recognized to have a strong tumor reducing effect. From this, it was verified that when BCG-CW is incorporated into an R8-modified liposome, a tumor reducing effect higher than that obtained in the case of directly administering BCG or BCG-CW, is obtained.

## Claims

1. A liposome having a lipid membrane containing a bacterial cell component which is insoluble in water and organic solvents.

2. The liposome according to claim 1, wherein the bacterial cell component is a cell wall fraction or cell wall skeleton fraction of a bacterium of genus Mycobacterium, a bacterium of genus Nocardia, a bacterium of genus Corynebacterium or a bacterium of genus Rhodococcus.

3. The liposome according to claim 2, wherein the bacterium of genus Mycobacterium is BCG.

4. The liposome according to any one of claims 1 to 3, having a peptide containing a plurality of contiguous arginine residues on the liposome surface.

5. The liposome according to claim 4, wherein the peptide is a peptide consisting of 4 to 35 amino acid residues including 4 to 20 contiguous arginine residues.

6. The liposome according to claim 4 or 5, wherein the peptide consists of arginine residues.

7. The liposome according to any one of claims 4 to 6, wherein the amount of the peptide is 2% (molar ratio) or more based on the total lipids constituting the liposome.

8. The liposome according to any one of claims 4 to 7, wherein the peptide is modified with a hydrophobic group or a hydrophobic compound, and the hydrophobic group or the hydrophobic compound is inserted into the lipid membrane forming the surface of the liposome, while the peptide is exposed from the lipid membrane forming the surface of the liposome.

9. A pharmaceutical composition comprising the liposome according to any one of claims 1 to 8.

10. The pharmaceutical composition according to claim 9, wherein the bacterial cell component has immuno activating potency, and the pharmaceutical composition is an immuno activator.
